# EUROPEAN PATENT APPLICATION

(11) **EP 2 606 905 A1**
(43) Date of publication of application: **26.06.2013**
(21) Application number: 11306766.4
(22) Date of filing: 23.12.2011
(51) Int. Cl.: A61K 38/04, C12N 15/11, C12N 5/10, A61P 19/00

(54) **Peptides targeting Receptor activator of nuclear factor-kappa B (RANK) and their applications**

(71) Applicant: Université de Nantes, 44000 Nantes (FR); CHU Nantes, 44000 Nantes (FR)
(72) Inventor: Heymann, Dominique, 44610 INDRE (FR); Teletchea, Stéphane, 44340 Bouguenais (FR); Stresing, Verena, 44000 NANTES (FR)
(74) Representative: Regimbeau

(57) **Abstract**

The present invention relates to a polypeptide for use as a medicament in the treatment and/or prevention of a disease wherein the RANKL-RANK signaling pathway is involved, in particular a bone resorptive disease.

SEQUENCE LISTING

<110>  UNIVERSITE DE NANTES
       CHU NANTES

<120>  PEPTIDES TARGETING RANK AND THEIR APPLICATIONS

<130>  69640D30383

<160>  45

<170>  PatentIn version 3.5

<210>  1
<211>  5
<212>  PRT
<213>  Artificial Sequence

<220>
<223>  Pep824 binding to the RANK cleft

<400>  1

Leu Lys Leu Cys Ser
1               5


<210>  2
<211>  9
<212>  PRT
<213>  Artificial Sequence

<220>
<223>  Pep8 binding to the RANK cleft

<400>  2

Asn Val Leu Lys Leu Cys Ser Gly Glu
1               5


<210>  3
<211>  12
<212>  PRT
<213>  Artificial Sequence

<220>
<223>  Pep801 binding to the RANK cleft

<400>  3

Glu Leu Ala Asn Val Leu Lys Leu Cys Ser Gly Glu
1               5                   10


<210>  4
<211>  11
<212>  PRT
<213>  Artificial Sequence

<220>
<223>  Pep802 binding to the RANK cleft

<400>  4

Asn Val Leu Lys Leu Cys Ser Gly Glu Ala Tyr
1               5                   10


<210>  5
<211>  14
<212>  PRT
<213>  Artificial Sequence

<220>
<223>  Pep803 binding to the RANK cleft

<400>  5

Glu Leu Ala Asn Val Leu Lys Leu Cys Ser Gly Glu Ala Tyr
1               5                   10


<210>  6
<211>  12
<212>  PRT
<213>  Artificial Sequence

<220>
<223>  Pep804 binding to the RANK cleft

<400>  6

Asn Val Leu Lys Leu Cys Ser Gly Glu Ala Tyr Arg
1               5                   10


<210>  7
<211>  10
<212>  PRT
<213>  Artificial Sequence

<220>
<223>  Pep805 binding to the RANK cleft

<400>  7

Asn Val Leu Lys Leu Ala Cys Ser Gly Glu
1               5                   10


<210>  8
<211>  8
<212>  PRT
<213>  Artificial Sequence

<220>
<223>  Pep806 binding to the RANK cleft

<400>  8

Asn Val Leu Lys Leu Cys Ser Glu
1               5


<210>  9
<211>  9
<212>  PRT
<213>  Artificial Sequence

<220>
<223>  Pep808 binding to the RANK cleft

<400>  9

Asn Val Leu Lys Phe Cys Ser Gly Glu
1               5


<210>  10
<211>  9
<212>  PRT
<213>  Artificial Sequence

<220>
<223>  Pep809 binding to the RANK cleft

<400>  10

Asn Val Ile Lys Leu Cys Ser Gly Glu
1               5


<210>  11
<211>  9
<212>  PRT
<213>  Artificial Sequence

<220>
<223>  Pep810 binding to the RANK cleft

<400>  11

Asn Val Leu Lys Leu Cys His Gly Glu
1               5


<210>  12
<211>  10
<212>  PRT
<213>  Artificial Sequence

<220>
<223>  Pep811 binding to the RANK cleft

<400>  12

Glu Asn Val Leu Lys Leu Cys Ser Gly Glu
1               5                   10


<210>  13
<211>  9
<212>  PRT
<213>  Artificial Sequence

<220>
<223>  Pep812 binding to the RANK cleft

<400>  13

Asn Ala Leu Lys Leu Cys Ser Gly Glu
1               5

<210>  14
<211>  9
<212>  PRT
<213>  Artificial Sequence

<220>
<223>  Pep813 binding to the RANK cleft

<400>  14

Glu Val Leu Lys Leu Cys Ser Gly Asn
1               5


<210>  15
<211>  9
<212>  PRT
<213>  Artificial Sequence

<220>
<223>  Pep814 binding to the RANK cleft

<400>  15

Asn Ala Leu Lys Phe Cys Ser Gly Glu
1               5


<210>  16
<211>  11
<212>  PRT
<213>  Artificial Sequence

<220>
<223>  Pep815 binding to the RANK cleft

<400>  16

Asn Ala Leu Lys Leu Cys Ser Gly Glu Met Arg
1               5                   10


<210>  17
<211>  10
<212>  PRT
<213>  Artificial Sequence

<220>
<223>  Pep816 binding to the RANK cleft

<400>  17

Asn Ala Leu Lys Leu Ala Cys Ser Gly Glu
1               5                   10


<210>  18
<211>  10
<212>  PRT
<213>  Artificial Sequence

<220>
<223>  Pep817 binding to the RANK cleft

<400>  18

Asn Ala Leu Lys Leu Phe Cys Ser Gly Glu
1               5                   10


<210>  19
<211>  12
<212>  PRT
<213>  Artificial Sequence

<220>
<223>  Pep818 binding to the RANK cleft

<400>  19

Asn Ala Leu Lys Leu Ala Cys Ser Gly Glu Met Arg
1               5                   10


<210>  20
<211>  9
<212>  PRT
<213>  Artificial Sequence

<220>
<223>  Pep819 binding to the RANK cleft

<400>  20

Asn Ala Leu Arg Leu Cys Ser Gly Glu
1               5


<210>  21
<211>  9
<212>  PRT
<213>  Artificial Sequence

<220>
<223>  Pep820 binding to the RANK cleft

<400>  21

Asn Ala Leu His Leu Cys Ser Gly Glu
1               5


<210>  22
<211>  9
<212>  PRT
<213>  Artificial Sequence

<220>
<223>  Pep821 binding to the RANK cleft

<400>  22

Asn Ala Leu Phe Leu Cys Ser Gly Glu
1               5


<210>  23
<211>  9
<212>  PRT
<213>  Artificial Sequence

<220>
<223>  Pep822 binding to the RANK cleft

<400>  23

Asn Ala Leu Asn Leu Cys Ser Gly Glu
1               5


<210>  24
<211>  13
<212>  PRT
<213>  Artificial Sequence

<220>
<223>  Pep825 binding to the RANK cleft

<400>  24

Tyr Cys Asn Val Leu Lys Leu Cys Ser Gly Glu Cys Tyr
1               5                   10


<210>  25
<211>  9
<212>  PRT
<213>  Artificial Sequence

<220>
<223>  Pep826 binding to the RANK cleft

<400>  25

Asn Ala Leu Lys His Cys Ser Gly Glu
1               5


<210>  26
<211>  12
<212>  PRT
<213>  Artificial Sequence

<220>
<223>  Pep501 binding to the RANK cleft

<400>  26

Glu Leu Ala Ser Phe Leu Lys Ile Ser Gln Leu Gly
1               5                   10


<210>  27
<211>  12
<212>  PRT
<213>  Artificial Sequence

<220>
<223>  Pep401 binding to the RANK cleft

<400>  27

Glu Leu Ala Ser Phe Asn Lys Ile Thr Gln Leu Gly
1               5                   10

<210>  28
<211>  12
<212>  PRT
<213>  Artificial Sequence

<220>
<223>  Pep402 binding to the RANK cleft

<400>  28

Glu Leu Ala Ser Phe Asn Arg Ile Thr Gln Leu Gly
1               5                   10


<210>  29
<211>  13
<212>  PRT
<213>  Artificial Sequence

<220>
<223>  PepA17 binding to the RANK cleft

<400>  29

Trp Leu Glu Thr Arg Leu Thr Asn His Met Glu Leu Gln
1               5                   10


<210>  30
<211>  13
<212>  PRT
<213>  Artificial Sequence

<220>
<223>  PepA18 binding to the RANK cleft

<400>  30

Ala Lys Phe His Gly Glu Leu Met Ala Asp Gln Trp Gln
1               5                   10


<210>  31
<211>  13
<212>  PRT
<213>  Artificial Sequence

<220>
<223>  PepA19 binding to the RANK cleft

<400>  31

Asn Glu Met Asp Leu Pro Lys Lys Ser Cys Leu Met Asn
1               5                   10


<210>  32
<211>  9
<212>  PRT
<213>  Artificial Sequence

<220>
<223>  PepA20 binding to the RANK cleft

<400>  32

Trp Ala Ala Arg Leu Gly Asp Pro Thr
1               5


<210>  33
<211>  12
<212>  PRT
<213>  Artificial Sequence

<220>
<223>  Pep1 binding to the RANK cleft

<400>  33

Glu Leu Ala Ser Tyr Ile Ile Ile Thr Gln Leu Gly
1               5                   10


<210>  34
<211>  21
<212>  DNA
<213>  Artificial Sequence

<220>
<223>  Cathepsin K forward primer

<400>  34
cccagactcc atcgactatc g                                                 21


<210>  35
<211>  24
<212>  DNA
<213>  Artificial Sequence

<220>
<223>  Cathepsin K reverse primer

<400>  35
ctgtaccctc tgcacttagc tgcc                                              24


<210>  36
<211>  21
<212>  DNA
<213>  Artificial Sequence

<220>
<223>  TRAP forward primer

<400>  36
aagactcact gggtggcttt g                                                 21


<210>  37
<211>  20
<212>  DNA
<213>  Artificial Sequence

<220>
<223>  TRAP reverse primer

<400>  37

ggcagtcatg ggagttcagg                                                    20

<210>  38
<211>  20
<212>  DNA
<213>  Artificial Sequence

<220>
<223>  NFATc1 forward primer

<400>  38
ggtcttcggg agaggagaaa                                                    20

<210>  39
<211>  20
<212>  DNA
<213>  Artificial Sequence

<220>
<223>  NFATc1 reverse primer

<400>  39
tgacgttgga ggatgcatag                                                    20

<210>  40
<211>  24
<212>  DNA
<213>  Artificial Sequence

<220>
<223>  GAPDH forward primer

<400>  40
tgggtgtgaa ccatgagaag tatg                                               24

<210>  41
<211>  18
<212>  DNA
<213>  Artificial Sequence

<220>
<223>  GAPDH reverse primer

<400>  41
ggtgcaggag gcattgct                                                      18

<210>  42
<211>  19
<212>  DNA
<213>  Artificial Sequence

<220>
<223>  B2M forward primer

<400>  42
ttctggcctg gaggctatc                                                     19

<210>  43
<211>  23

<212>  DNA
<213>  Artificial Sequence

<220>
<223>  B2M reverse primer

<400>  43
tcaggaaatt tgactttcca ttc                                               23


<210>  44
<211>  18
<212>  DNA
<213>  Artificial Sequence

<220>
<223>  ACTB forward primer

<400>  44
ccaaccgcga gaagatga                                                     18


<210>  45
<211>  20
<212>  DNA
<213>  Artificial Sequence

<220>
<223>  ACTB reverse primer

<400>  45
ccagaggcgt acagggatag                                                   20

## Description

The present invention relates to the field of prevention and treatment of diseases related to activation of the RANKL-RANK signaling pathway.

In particular, the invention relates to an isolated polypeptide useful for the prevention and treatment of bone resorptive diseases.

Bone tissue undergoes constant remodeling to fulfill its principal functions of mechanical support, maintenance of calcium homeostasis, and as a stem cell supplier. This process is mediated by two cell lineages: the hematopoietic bone-resorbing osteoclasts and the bone-forming mesenchymal osteoblasts and osteocytes. Under physiological conditions, the balance between bone formation and resorption is tightly regulated and determines bone density. This balance is based on the RANK/RANKL/OPG pathway.

Receptor activator of nuclear factor-κB (RANK) is a member of the tumor necrosis factor family expressed by osteoclasts and their precursors. The interaction of RANK with RANKL (its ligand) has been identified as the final common pathway through which bone resorption is regulated. By binding to its receptor RANK on osteoclastic precursors, RANKL controls the differentiation, proliferation, and survival of osteoclasts. Osteoprotegerin (OPG) is the natural inhibitor of RANKL. RANK and RANKL are expressed in many regular cell types (Theoleyre *et al.* 2004) but their activity is the most prominent in bone tissue, skin (Duheron *et al.* 2011) and mammary glands (Gonzalez-Suarez et al 2010; Schramek et al 2010). RANK-RANKL expression is severely enhanced in non-bone cancer cells such as breast or melanoma cancers, in their associated metastasis (Jones *et al.* 2006) and in primary bone cancers as in osteosarcoma (Mori *et al.* 2007).

Disruption of the homeostatic balance can lead to pathologic bone loss, such as in age-related osteoporosis, periodontal disease or inflammatory rheumatoid arthritis, or to excessive bone formation, such as in skeletal malformations linked or not with genetic mutations and/or polymorphisms (Whyte *et al.* 2009) or to alteration in bone remodeling. Perturbations in the ratio of OPG to RANKL have been demonstrated to occur with estrogen deficiency, hyperparathyroidism, and other disorders that stimulate bone resorption. RANKL is also expressed by lymphocytes and synovial fibroblasts and may mediate bone loss associated with inflammatory conditions.

The discovery of the RANK/RANKL/OPG pathway and its implications in the pathogenesis of bone diseases provided a molecular target for therapies to improve bone health.

The development of small molecules, OPG mimetics which target RANKL have been suggested for the development of therapeutic agents to treat bone diseases, in particular bone resorptive diseases. Cheng and his co-workers (Cheng *et al.* 2004) have designed such peptides derived from OPG to block RANKL. Their most promising peptide OP3-4 was able to directly bind RANKL with a measurable interaction by surface plasmon resonance. This interaction was sufficient to reduce *in vitro* osteoclastogenesis and protect mice *in vivo* from bone loss.

Another approach initiated by Takasaki in 1997 (Takasaki *et al.* 1997) was to design bio-compatible molecules able to target RANKL based on peptides derived from the RANK sequence. Their work, initially targeting the TNF-alpha/TNFR interaction, proved useful to block the RANKL/RANK interaction in a TNF-independent way (Aoki *et al.* 2006). Their most effective peptide named WP9QY was able to inhibit *in vitro* osteoclastogenesis in a dose-dependent manner and prevent *in vivo* bone loss in a mouse osteoporosis-induced model.

These peptides were derived from the native sequence of the partner they were targeting, based on assumptions made upon models based on other members of the TNF/TNF-R family: *i.e.* OP3-4 was selected from the putative OPG-RANKL interface (the model for OPG used TNFR, Fas and TRAIL crystallographic structures) and WP9QY was selected from the putative RANKL-RANK interface (models were based on the TNF-R/TNF-β crystallographic structure). Although their biologic activity showed promising results in *in vitro* and *in vivo* models, their therapeutic interest was potentially limited by their relatively low binding affinity to RANKL, in comparison to the binding of RANKL to RANK.

Over the past decade, there have been tremendous advances in the management of metabolic bone disorders with the introduction of novel bone-chelating agents (zoledronate being the most prominent treatment to date) and the development of a monoclonal antibody (denosumab) directed against RANKL (Baron *et al.* 2011). Although these therapies show promising improvements in the treatment of bone diseases, in particular in bone resorptive diseases, their application is limited by the poor bioavailability and/or stability of large macromolecules, such as antibodies or chimeras, the mode of administration, the high cost and the risk of mild to severe and sometimes even life-compromising side effects, such as skin rashes, immunogenicity, osteonecrosis of the jaw or increased trabecular bone mineral density leading to growth retardation.

There remains, therefore, a significant need for new and improved compounds for the prevention and/or treatment of bone diseases, in particular of bone resorptive diseases which are effective in inhibiting osteoclastogenesis, cheap to produce, possess a high bioavailability, that may be easily administered to patients while being without severe side effects. The present inventors have made a significant step forward with the invention disclosed herein.

The purpose of the invention is to fulfill this need by providing new polypeptides, which make it possible to solve in whole or part the problems mentioned above.

The inventors have designed innovative peptides which are able to specifically bind the receptor RANK and have a strong inhibitory effect on osteoclastogenesis. Contrary to the peptides developed so far, the peptide sequences of the invention are not derived from the existing RANK, RANKL or OPG amino acid sequences and are not present in any natural protein or peptide known in the literature.

In one aspect, the invention relates to an isolated polypeptide having a sequence of up to 20 amino acids, wherein said sequence comprising or consisting of a sequence selected from the group consisting of:
- SEQ ID NO: 1 (LKLCS); and
- a sequence having at least 80% of identity with SEQ ID NO: 1 over the entire length of SEQ ID NO: 1;
for use as a medicament in the treatment and/or prevention of a bone disease, in particular a bone resorptive disease.

The polypeptide according to the invention has in particular the following advantages:
- It has a strong inhibitory effect on osteoclastogenesis;
- It has no severe side effects. In particular, it has no obvious cytotoxicity or pro-apoptotic activities;
- It is cheap to produce;
- It possesses a high bioavailability;
- It has a high capacity to penetrate tumors. As illustrated in the article of Lien *et al.* (2003), it is well known in the art that in the treatment of cancer, a further advantage of peptides over larger proteins such as full length antibodies is their superior ability to penetrate tumors;
- It proved to be very stable over time which possibly elicits its administration to patients via any existing delivery technique.

Unless defined otherwise, all technical and scientific terms used herein have the same meaning as commonly understood to one skilled in the relevant art.

For convenience, the meaning of certain terms and phrases employed in the specification, examples and claims are provided.

As used herein, the term "bone resorptive disease" refers to any disease wherein bone homeostasis is altered through the RANKL-RANK signaling pathway. The homeostasis imbalance may appear in non-inflammatory or in inflammatory bone resorptive diseases, in oncologic or in non-oncologic bone resorptive diseases as described in the article of Theoleyre *et al.* (2004).

In a non-inflammatory context, bone resorptive diseases include metabolic bone diseases such as osteoporosis (including type I (postmenopausal) and type II (senile) osteoporosis), skeletal malformations linked or not with genetic mutations and/or polymorphisms (Whyte *et al.* 2009), vitamin D deficiency such as observed in rachitism, any disease caused by any treatment having as a side effect altered bone remodeling (including endocrine therapies such as tamoxifen or aromatase inhibitors (Lee *et al.* 2011), glucocorticoid-induced osteoporosis (Hansen *et al.* 2011), hypercalcemia of malignancy), osteolytic bone diseases (benign tumors such as multiple myeloma or giant-cell tumor of bone (*Croucher et al. 2001*) or bone sarcomas (Dai *et al.* 2011)).

Postmenopausal osteoporosis (also called PMO or type I osteoporosis) is primarily due to estrogen deficiency.

Senile osteoporosis (also called type II osteoporosis) is primarily due to an aging skeleton and calcium deficiency.

Glucocorticoid-induced osteoporosis is a form of osteoporosis that is caused by taking glucocorticoid medications such as prednisone (Deltasone, Orasone, etc.), prednisolone (Prelone), dexamethasone (Decadron, Hexadrol), and cortisone (Cortone Acetate).

In an inflammatory context, bone resorptive diseases include all arthritis pathologies such as rheumatoid arthritis, osteolytic bone diseases, periodontal diseases and any cardiovascular disease wherein the RANKL-RANK pathway is involved such as atherosclerosis and fractures.

In the oncologic context, bone resorptive diseases include:
- primary bone cancers such as osteosarcoma, Ewing's sarcoma, chondrosarcoma, and benign bone cancers such as multiple myeloma or giant-cell bone tumor (Dai *et al.* 2011; Croucher *et al.* 2001);
- primary cancers wherein the RANKL-RANK signaling pathway is a direct enhancer of the tumor growth, such as breast cancer (Jones *et al.* 2006; Schramek *et al.* 2010; Gonzalez-Suarez 2010), multiple myeloma (Demchenko and Kuehl 2010), carcinoma, neuroblastoma, chondroblastoma, colorectal cancer, renal cancer, esophageal cancer, hepatic cancer, cervical cancer, endometrial cancer (Theoleyre *et al.* 2004; Santini *et al.* 2011);
- secondary bone cancers also described as skeletal metastasis, following a primary cancer including prostate cancer, breast cancer (Schramek *et al.* 2010; Gonzalez-Suarez 2010), lung cancer, colorectal cancer, renal cancer, esophageal cancer, bladder cancer, hepatic cancer, cervical cancer, endometrial cancer, salivary glands cancer, squamous cancer, malignant melanoma (Smith 2011; Santini *et al.* 2011)

As used herein, the term "primary bone cancer" refers to any cancer which originates in a bone. Primary bone cancers include but are not limited to osteosarcoma (also called osteogenic sarcoma), Ewing's sarcoma, chondrosarcoma.

As used herein, the term "secondary bone cancer" refers to any skeletal metastasis following a primary cancer including prostate cancer, breast cancer, lung cancer, colorectal cancer, renal cancer, esophageal cancer, bladder cancer, hepatic cancer, cervical cancer, endometrial cancer, salivary glands cancer, squamous cancer, malignant melanoma. Secondary bone cancer is the result of cancer cells spreading to the bone from a primary tumor.

In particular, bone resorptive diseases are selected from the group consisting of osteoporosis, osteolytic bone disease, primary bone cancers, secondary bone cancers, periodontal disease and rheumatoid arthritis.

More particularly, bone diseases are selected from the group consisting of osteoporosis, primary bone cancers, secondary bone cancers and rheumatoid arthritis.

In particular, the isolated polypeptide according to the invention has a sequence of up to 19 amino acids, more particularly of up to 18, 17, 16, 15, 14, 13, 12, 11, 10, 9, 8, 7, 6, 5, 4 and even more particularly of up to 9, 8, 7, 6, 5, 4 amino acids.

In particular, the isolated polypeptide according to the invention, more particularly the isolated polypeptide which have a sequence comprising or consisting of a sequence having at least 80% of identity with SEQ ID NO: 1 over the entire length of SEQ ID NO: 1, have the ability to inhibit RANKL-induced osteoclastogenesis.

The ability of a polypeptide to inhibit RANKL-induced osteoclastogenesis can be determined by one skilled in the art by tests including assessment of the effect of polypeptide on osteoclast differentiation as described in the articles of Baud'Huin *et al.* 2009 and Duplomb *et al.* 2008.

In particular, the isolated polypeptide according to the invention have a sequence comprising or consisting of a sequence having at least 80%, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with SEQ ID NO: 1 over the entire length of SEQ ID NO: 1.

The percentages of identity to which reference is made in the presentation of the present invention are determined on the basis of a global alignment of sequences to be compared, that is to say, on an alignment of sequences over their entire length, using for example the algorithm of Needleman and Wunsch 1970. This sequence comparison can be done for example using the needle software by using the parameter "Gap open" equal to 10.0, the parameter "Gap Extend" equal to 0.5, and a matrix "BLOSUM 62". Software such as needle is available on the website ebi.ac.uk worldwide, under the name "needle".

In particular, the isolated polypeptide according to the invention has a sequence comprising or consisting of a sequence selected from the group consisting of:
- SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25, more particularly SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14, even more particularly SEQ ID NO: 1, SEQ ID NO: 2, SEQ ID NO: 4, SEQ ID NO: 6, SEQ ID NO: 7, SEQ ID NO: 8, SEQ ID NO: 9, SEQ ID NO: 12 and SEQ ID NO: 13, even more particularly SEQ ID NO: 1, SEQ ID NO: 2 and SEQ ID NO: 6 and even more particularly SEQ ID NO: 1 and SEQ ID NO: 2.

In particular, the isolated polypeptide according to the invention has a sequence consisting of a sequence selected from the group consisting of:
SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25, more particularly SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14, even more particularly SEQ ID NO: 1, SEQ ID NO: 2, SEQ ID NO: 4, SEQ ID NO: 6, SEQ ID NO: 7, SEQ ID NO: 8, SEQ ID NO: 9, SEQ ID NO: 12 and SEQ ID NO: 13, even more particularly SEQ ID NO: 1, SEQ ID NO: 2 and SEQ ID NO: 6 and even more particularly SEQ ID NO: 2.

In particular, the isolated polypeptide according to the invention contains at least one biochemical modification selected from the group consisting of pegylation, acetylation, formylation, myristic acid addition, palmytoylation, benzyloxycarbonylation, amidation, succinylation, glycosylation, in particular pegylation.

In particular, the isolated polypeptide according to the invention comprises at least one polyethylene glycol group, more particularly at least 2, at least 3, at least 4, at least 5, at least 6, at least 7, at least 8, at least 9, at least 10, polyethylene glycol groups, in a linear, branched or more complex organization.

The pegylation of the polypeptide of the invention has the advantage that it optimizes the polypeptide solubility, bioavailability and stability and decrease the immunogenicity.

In particular, the isolated polypeptide according to the invention comprises less than 20 polyethylene glycol groups, more particularly less than 19, less than 18, less than 17, less than 16, less than 15, less than 14, less than 13, less than 12 and even more particularly less than 11 polyethylene glycol groups.

In particular, the isolated polypeptide according to the invention comprises 1 polyethylene glycol group per amino acid.

Said polyethylene group can be attached to any appropriate amino acid suitable for modification of the polypeptide of the invention. In particular, at least one polyethylene glycol group is attached to the N-terminal amino acid or to the C-terminal of said polypeptide. More particularly, at least one polyethylene glycol group is attached to the N-terminal amino acid of said polypeptide.

The invention also relates to an isolated polypeptide according to the invention as described above. The advantageous embodiments are as defined above.

In particular, the isolated polypeptide according to the invention has the amino acid sequence set forth in SEQ ID NO: 2.

The present invention encompasses polypeptides as described above having modified amino acid sequences. Modifications can include but are not limited to amino acid insertions, deletions, substitutions, truncations, fusions, cyclization, disulfide bridging, substitution of D-amino acids by L-amino acids or by beta peptides, modifications to improve cell-membrane pass-through (for instance using a signal peptide or a fragment of Antennapedia homeodomain, Derossi *et al.* 1994; May *et al.* 2000) provided that the polypeptides retain the ability to inhibit RANKL-induced osteoclastogenesis. Such modifications may be undertaken to improve polypeptide half-life or biological activity.

The polypeptides according to the invention can be synthesized using conventional methods including chemical synthesis and synthesis using nucleic acid molecules encoding said polypeptides.

The invention also relates to an isolated nucleic acid molecule encoding an isolated polypeptide according to the invention.

In particular, the nucleic acid molecule according to the invention can be operatively linked to a promoter for a eukaryotic DNA dependent RNA polymerase, preferably for RNA polymerase II. If tissue-specific RNA polymerase II promoters are used, the polypeptide of the invention can be selectively expressed in the targeted tissues/cells.

Said promoter can be a constitutive promoter or an inducible promoter well known by one skilled in the art. The promoter can be developmentally regulated, inducible or tissue specific.

In particular, the nucleic acid molecule according to the invention can be operatively linked to an extracellular signal sequence. Said extracellular signal sequence can encode a signal peptide allowing the secretion of the polypeptide of the invention in the extracellular medium. Extracellular signal sequences are well known by one skilled in the art and can encode as an example e the signal peptide having the following sequence "MAPRARRRRPLFALLLLCALLARLQVALQ" (hRANK signal peptide) (Petersen *et al.* 2011).

The invention also relates to a vector comprising a nucleic acid molecule according to the invention. Said vector can be appropriated for semi-stable or stable expression.

Particularly, said vector according to the invention is a cloning or an expression vector.

The vectors can be viral vectors such as bacteriophages or non-viral such as plasmids.

The invention also relates to a host cell comprising a nucleic acid molecule according to the invention or a vector according to the invention.

The host cell according to the invention can be useful for production of a polypeptide according to the invention.

The invention also relates to a pharmaceutical composition comprising at least one compound selected from the group consisting of an isolated polypeptide according to the invention, an isolated nucleic acid molecule according to the invention and a vector according to the invention. The advantageous embodiments are as defined above.

The terms "medicament" and "pharmaceutical composition" are used interchangeably and in their broadest sense herein.

Such compound (in particular selected from the group consisting of an isolated polypeptide according to the invention, an isolated nucleic acid molecule according to the invention and a vector according to the invention) can be present in the pharmaceutical composition according to the invention in a therapeutically effective amount (active and nontoxic amount). A therapeutically effective amount refers to that amount of compound which ameliorates the symptoms or condition. Therapeutic efficacy and toxicity may be determined by standard pharmaceutical procedures in cell cultures or experimental animals, e.g., ED50 (the amount therapeutically effective in 50% of the population) and LD50 (the dose lethal to 50% of the population). The amount ratio of toxic to therapeutic effects is the therapeutic index and it can be expressed as the ratio, LD50/ED50. Pharmaceutical compositions which exhibit large therapeutic indices are preferred.

For example, the polypeptide according to the invention, particularly the polypeptide having a sequence set forth in SEQ ID NO: 2, can be administered to a patient, in particular by injection, in an amount within the range from 0.1 to 100 mg/kg of body weight of said patient daily, particularly within the range from 0.5 to 50 mg/kg of body weight of said patient daily and even more particularly within the range from 0.5 to 10 mg/kg of body weight of said patient daily. Said doses will be adjusted to elicit a therapeutic response yet able to protect from a potential immunogenicity of the peptide (Toes *et al.* 1998).

The pharmaceutical composition according to the invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

In addition to the active ingredients, the pharmaceutical composition of the invention may contain suitable pharmaceutically acceptable carriers comprising excipients and auxiliaries, which facilitate processing of the active compounds into preparations which can be used pharmaceutically. In particular, the pharmaceutical composition according to the invention is formulated in a pharmaceutical acceptable carrier. Pharmaceutical acceptable carriers are well known by one skilled in the art. Further details on techniques for formulation and administration may be found in the latest edition of Remington's Pharmaceutical Sciences (Maack Publishing Co., Easton, Pa.).

In particular, the pharmaceutical acceptable carrier is a biomaterial.

As used herein the term "biomaterial" refers to any material that is biocompatible, in particular designed to interact with biological systems.

The biomaterial has the advantage that it may precisely determine the initial delivery zone of the polypeptide of the invention, in particular the polypeptide having a sequence set forth in SEQ ID NO: 2, in order to enhance its local efficacy and bioavailability, in a time and/or dose controlled way.

The biomaterial can be selected from the group consisting of phosphocalcic ceramics (e.g. hydroxyapatite (HAP) and beta-tricalcium phosphate), polymers (e.g. copolymers of lactic acid and glycolic acid, hydrogels), materials of natural origin (e.g. cellulose, collagen), particularly phosphocalcic ceramics and even more particularly phosphocalcic ceramics consisting of 40% beta-tricalcium phosphate and 60% hydroxyapatite.

Hydroxyapatite (HAP) and tricalciumphosphate (in particular beta-tricalcium phosphate) ceramics have the advantage to be bioresorbable and osteoconductive.

Pharmaceutical composition suitable for parenteral administration may be formulated in aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

The invention also relates to a method for the treatment and/or prevention of a bone resorptive disease comprising the step of administering to a patient in need thereof a therapeutically effective amount of at least one compound selected from the group consisting of an isolated polypeptide according to the invention, an isolated nucleic acid molecule according to the invention and a vector according to the invention. The advantageous embodiments are as defined above.

The invention also relates to a method of inhibiting osteoclastogenesis comprising the step of administering to a patient in need thereof, in particular to a patient having a bone disease and more particularly to a patient having a bone resorptive disease, a therapeutically effective amount of at least one compound selected from the group consisting of an isolated polypeptide according to the invention, an isolated nucleic acid molecule according to the invention and a vector according to the invention. The advantageous embodiments are as defined above.

The invention also relates to an isolated polypeptide according to the invention for use as a medicament. The advantageous embodiments are as defined above.

The invention also relates to an isolated nucleic acid molecule according to the invention for use as a medicament. The advantageous embodiments are as defined above.

The invention also relates to a vector according to the invention for use as a medicament. The advantageous embodiments are as defined above.

The invention also relates to the use of an isolated polypeptide according to the invention for the preparation of a medicament for the treatment and/or prevention of a bone disease, in particular a bone resorptive disease. The advantageous embodiments are as defined above.

The invention also relates to the use of an isolated nucleic acid molecule according to the invention for the preparation of a medicament for the treatment and/or prevention of a bone disease, in particular a bone resorptive disease. The advantageous embodiments are as defined above.

The invention also relates to the use of a vector according to the invention for the preparation of a medicament for the treatment and/or prevention of a bone disease, in particular a bone resorptive disease. The advantageous embodiments are as defined above.

The invention also relates to a combination product comprising:
- at least one compound selected from the group consisting of an isolated polypeptide according to the invention, an isolated nucleic acid molecule according to the invention and a vector according to the invention; and
- another bone anti-resorptive agent,
for simultaneous, separate or sequential use as a medicament.

The advantageous embodiments are as defined above.

As used herein the term "bone anti-resorptive agent" refers to any agent able to inhibit bone-resorption.

In particular, said another bone anti-resorptive agent is selected from the group consisting of:
- anabolism enhancers, in particular selected from the group consisting of parathyroid hormone, BMP2, vitamin D, prostaglandin E2, anti-inflammatory agents; and
- catabolism inhibitors, in particular selected from the group consisting of bisphosphonates, cathepsin K inhibitors, p38 inhibitors, JNK inhibitors, IKK inhibitors, NF-κB inhibitors, calcineurin inhibitors, NFAT inhibitor, PI3K inhibitor (Tanaka *et al.* 2005).

As used herein, the term "catabolism inhibitors" refers to any agent able to protect from bone destruction (Xu *et al.* 2010).

As used herein, the term "anabolism enhancers" refers to any agent able to restore normal bone mineral density (Boyce *et al.* 2006).

The invention also relates to a combination product comprising:
- at least one compound selected from the group consisting of an isolated polypeptide according to the invention, an isolated nucleic acid molecule according to the invention and a vector according to the invention; and
- an anti-tumoral agent,
for simultaneous, separate or sequential use as a medicament.

As used herein, the term « anti-tumoral agent » refers to any agent able to prevent and/or treat a cancer, alone or in combination with another agent.

In particular, said anti-tumoral agent is able to prevent and/or treat a primary bone cancer and/or a secondary bone cancer.

In particular, said anti-tumoral agent can be selected in the group consisting of:
- actinomycin-D, bleomycin, cisplatin, cyclophosphamide, dactinomycin, doxorubicin, etoposide, gemcitabine, ifosfamide, methotrexate (and highdose methotrexate with leucovorin calcium rescue), paclitaxel, vincristine (Dai *et al.* 2011; Wittig *et al.* 2002).

The invention also relates to a combination product according to the invention, for its use in the treatment and/or prevention of a bone resorptive disease, in particular a bone resorptive disease selected from the group consisting of:
- osteoporosis, osteolytic bone disease, primary bone cancers, secondary bone cancers, periodontal disease and rheumatoid arthritis.

The advantageous embodiments are as defined above.

### BRIEF DESCRIPTION OF DRAWINGS

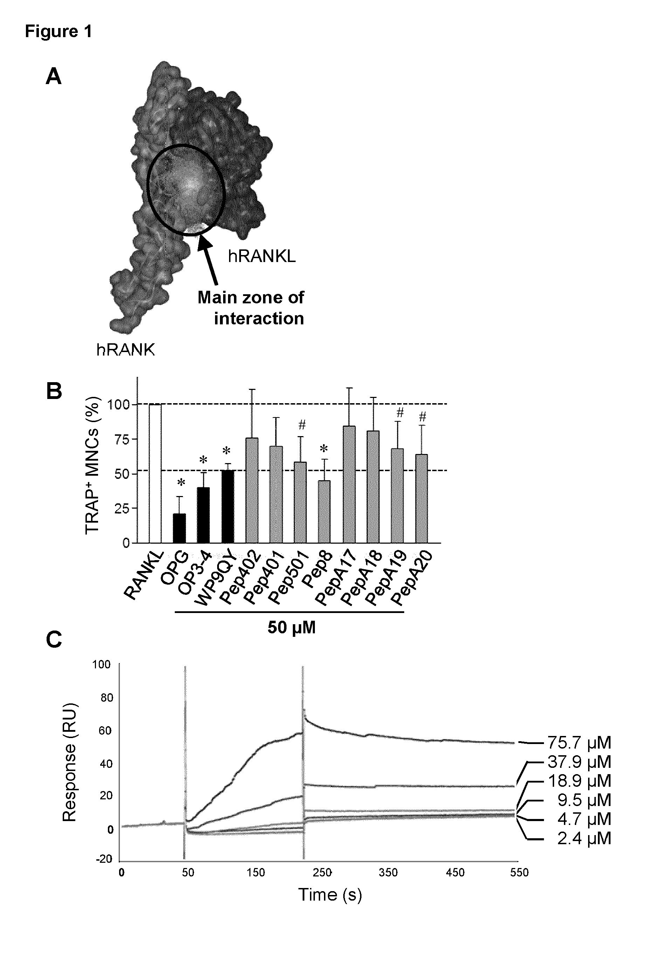

**Figure 1** **(A-C)** represents the development of small polypeptides inhibiting RANK/RANKL activity. **(A)** Three-dimensional structure of the RANK-RANKL dimer and main zone of polypeptide interaction from molecular modeling. **(B)** Effect of inhibitor polypeptide targeting RANK on osteoclast differentiation. CD14+ monocytes were treated with M-SCF (25 ng/ml) and human RANKL (100 ng/ml) in the presence or absence of each polypeptide (50 µM). When osteoclasts had formed, cells were fixed and stained for TRAP, and the TRAP+ multinucleated cells (MNCs) were counted. Results are expressed as percentage of TRAP+ cells in cultures without the corresponding polypeptide. The RANKL-targeting polypeptides WP9QY and OP3-4, as well as the RANKL decoy receptor OPG (50 ng/ml) were used as positive controls. Results are expressed as means ± SD of three experiments carried out in triplicate. *P < 0.005 and #P < 0.05. **(C)** The affinity of polypeptide binding to RANK (here, Pep1 (SEQ ID NO: 33)) was measured by Surface plasmon resonance (Biacore) analysis. The sensorgram shows the relative response in resonance units after background subtraction vs. time in seconds. Binding of Pep1 to hRANK is shown at concentrations indicated in the graph. Pep1 binds RANK with a K_{d} of 20,6 µM (kₒₙ = 1,35.10¹ M⁻¹s⁻¹, k_{off} = 2,78.10⁻⁴ s⁻¹).

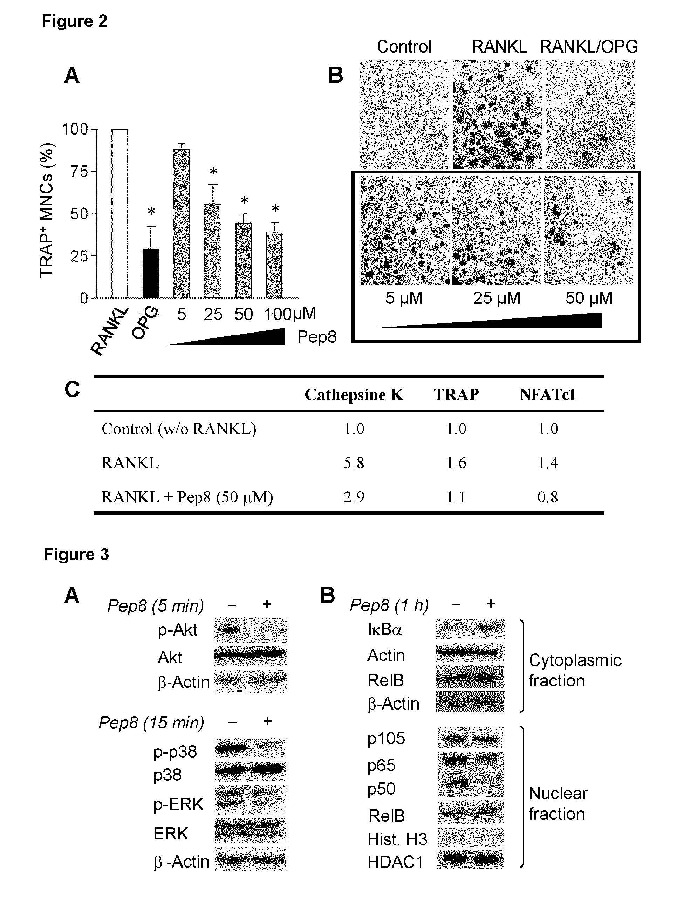

**Figure 2** **(A-C)** shows the inhibition of RANKL-induced osteoclastogenesis *in vitro* by Pep 8 (SEQ ID NO: 2). **(A)** Human monocytes were cultured with M-CSF and human RANKL in the presence or absence of increasing concentrations of Pep8, as indicated. OPG (50 ng/ml) was used as a reference inhibitor. Pep8 dose-dependently inhibited osteoclast formation in CD14⁺ cells. Results are expressed as means ± SD of at least two independent experiments carried out in triplicate versus treated with RANKL and M-SCF in the absence of Pep8 (RANKL). **P* < 0.05. **(B)** Representative microscopic images of TRAP⁺ cells at different concentrations of Pep8. **(C)** Real-time quantitative RT-PCR analysis for relative expression of osteoclast-specific genes, cathepsin K, TRAP and nuclear factor of activated T cells c1 (NFATc1) showed that Pep8 inhibited the mRNA expression of cathepsin K, TRAP and NFATc1. CD14⁺ cells were treated with M-SCF and sRANKL for 24h in the presence or absence of Pep8 (50 µM). Levels of the markers were normalized to beta-2-microglobulin (B2M). Expression level of undifferentiated cells (control) was set to 1. At least three independent experiments per marker were carried out. The table shows the results of a representative experiment.
**Figure 3** **(A-B)** shows the inhibition of RANKL-induced signaling by Pep8. **(A)** Inhibition of activation of Akt, p38 and ERK by Pep8 (50 µM) was assessed as inhibition of phosphorylation (p) in RAW264.7 cells using Western blot analysis with antibodies against phospho-Akt, phospho-p38 and phospho-ERK. Immunoblots were stripped and then reprobed with antibodies against Akt, p38 and ERK. β-Actin served as loading control. **(B)** Western blot analyses of NF-κB distribution in the presence or absence of 50 µM Pep8. After treatment with the polypeptide, RAW264.7 cells were fractionated into nuclear and cytoplasmic portions. Nuclear proteins (45 µg) were analyzed with anti-NF-κB p105, p50 and p65 antibodies, while cytoplasmic proteins (30 µg) were analyzed with anti-IκBα. For the non-canonical NF-κB pathway, RelB expression was analyzed in both fractions. β-Actin, HDAC1 and Histone H3 served as loading controls for the cytoplasmic and nuclear fractions, respectively. A representative experiment is shown, time after RANKL stimulation is indicated in parenthesis.

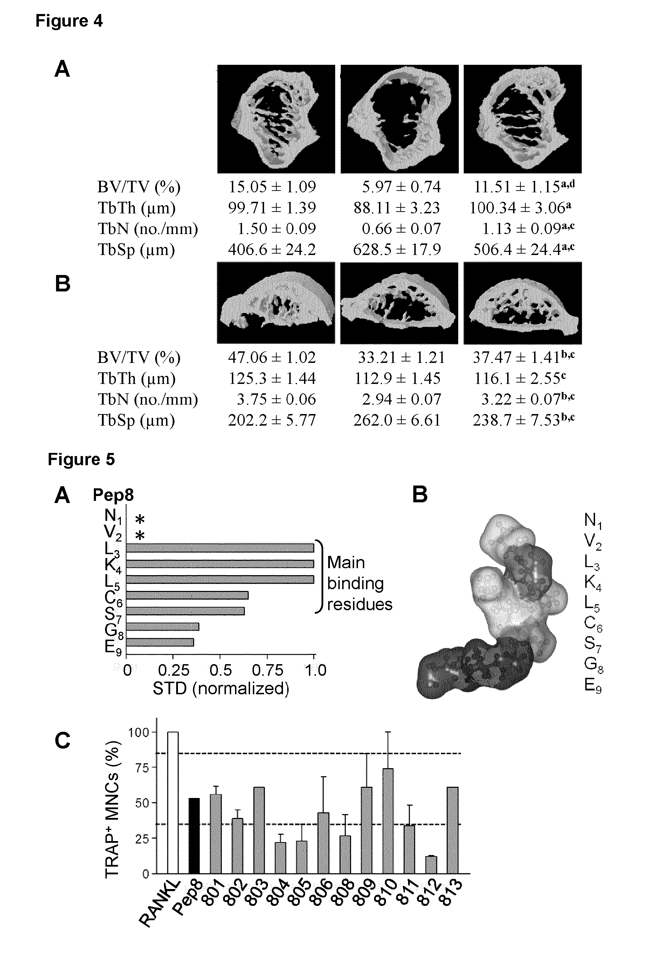

**Figure 4** **(A-B)** shows the inhibition of ovariectomy-induced bone loss by Pep8. The effect of Pep8 on *in vivo* bone density was determined in ovariectomized mice. 8-week-old C57BL/6 mice were ovariectomized and treated for 35 days with 10 mg/kg/day Pep8 or vehicle. Non-ovariectomized mice treated with the vehicle were included as healthy controls. The 3-dimensional structure of the trabecular bone in tibiae **(A)** and 4^{th} lumbar vertebrae **(B)** was examined by micro-computed tomography (µCT), as described in Methods. The amount of trabecular bone in OVX mice was markedly less than the amount of trabecular bone in healthy controls (NOV). Treatment with Pep8 (10 mg/kg) prevented the loss of trabecular bone. Histomorphometric analysis shows a significant increase in bone volume (BV)/tissue volume (TV) ratio, trabecular thickness (TbTh), trabecular number (TbN), and decrease in trabecular spacing (TbS) in mice treated with Pep8. Values are the mean ± SEM. ^{a}*P* < 0.005 and ^{b}*P* < 0.05 vs. OVX; ^{c}*P* < 0.005 and ^{d}*P* < 0.05 vs. NOV.
**Figure 5** **(A-C)** illustrates the characterization of the main binding residues and the inhibitory activity of a series of polypeptides of the invention on osteoclastogenesis. **(A)** Saturation transfer difference (STD) of Pep8 binding to RANK. The central motif Leu-Lys-Leu-Cys-Ser (L₃K₄L₅C₆S₇) receives the most important part of the transfer, which is in agreement with the predicted binding zone defined by molecular modeling. Due to experimental limitations, amino acids N₁ (Asp) and V₂ (Val) are not seen in the experiment. Both NH-groups from amino acid N₁ are detected, but show lower saturation transfers than NH from G₈ (Gly) and E₉ (Glu). **(B)** Three-dimensional conformation of Pep8 bound to RANK from molecular modeling. **(C)** Inhibitory activity of a series of polypeptides of the invention (series 800) on osteoclastogenesis. The polypeptides are derivatives of Pep8, which were generated based on molecular modeling results of specific binding affinities of key amino acids to RANK

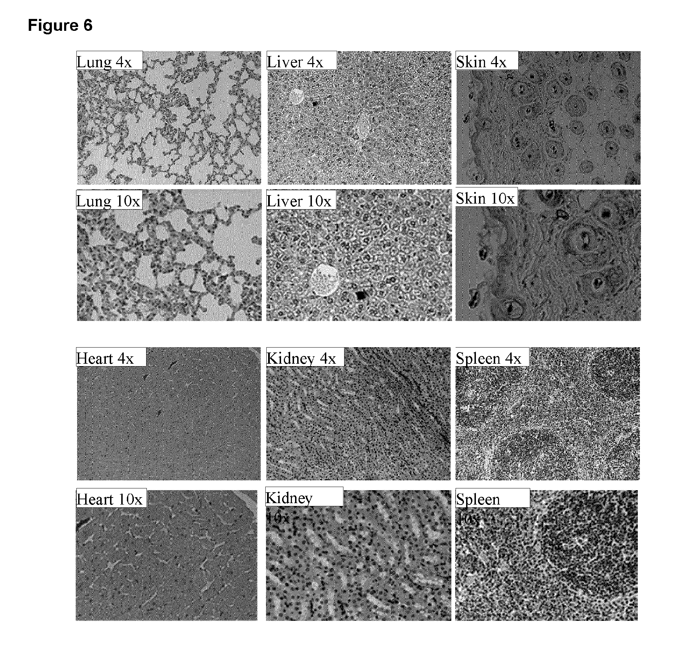

**Figure 6** represents the histomorphological evaluation of organ toxicity after treatment with Pep8 for 5 weeks. No histomophological difference was observed compared to control groups.

The present invention will be explained in detail with examples in the following text, but the technical scope of the present invention is not limited to these examples.

### EXAMPLES

### I. Material and Methods

### I.1 Generation of a large collection of polypeptides

A wide database of polypeptides filtered for aqueous solubility was generated using in-house bioinformatics tools. Briefly, random amino acids sequences of desired length (7-13 amino acids) were generated *in silico* and the resulting polypeptide sequences were filtered according to a combination of biochemical and sequence-related filters to ensure most resulting polypeptides will be soluble *in vitro* and *in vivo* with most common solvents (H₂O, PBS, DMSO/H₂O). The resulting collection was processed to provide the three-dimensional coordinates of the polypeptide. Each polypeptide was further typed with the CHARMm force-field (Brooks *et al.* 1983) to be suitable for further analysis within Discovery Studio 2.5.5 (Accelrys Software Inc, San Diego, CA, USA).

### I.2 Docking experiments and refinement of docking poses

The Human RANK-RANKL crystal structure (PDB id: 3ME2, Liu *et al.* 2010) was used as a reference to define putative binding sites at the RANK-RANKL interaction site. The binding of RANKL induces a 36° switch of the two cysteine-rich domains (CRD) in N-terminal of RANK (Liu *et al.* 2010). The inventors defined the hinge region between the first two and the last two CRDs on RANK as the most favorable binding pocket for docking studies since they contain critical contacts for RANKL-binding (Ta *et al.* 2010). Docking experiments were performed using the CDOCKER module (Wu *et al.* 2003) of Discovery Studio 2.5.5. Principal poses were visually inspected and the most promising poses were refined for a better characterization of the most favorable RANK-polypeptide interactions.

### I.3 Surface Plasmon Resonance Binding Assay

Biosensor experiments were carried out on a BIAcore 3000 instrument (BIAcore) as published previously (Baud'Huin *et al.* 2009). Recombinant purified RANKL (2 µg/mL in 5 mM maleate, pH 5.75) or RANK-Fc carrier-free (5 µg/mL in sodium acetate buffer, pH 5.0) were covalently immobilized to the dextran matrix of a CM5 sensor chip (BIAcore) at a flow rate of 5 µL/min. Immobilization levels ranging from 400 to 3000 Response Units (RU) for RANKL or 5000 RU for RANK were obtained. Binding assays were performed at 25°C in 10 mM Hepes buffer, pH 7.4. The association phase was 180 seconds followed by a dissociation phase in the same buffer. Polypeptides binding studies to RANK or RANKL were determined using single cycle kinetics, starting with 100 µM of the polypeptide of interest followed by 2-fold serial dilutions (ranging from 100 to 0.78 µM). The sensorgrams were fitted to calculate the equilibrium-dissociation constants using the Langmuir 1:1 model with BiaEval 4.1 software (BIAcore). RANK-Fc CF, RANKL and OPG carrier-free were obtained from R&D Systems (Minneapolis, MN, USA).

### I.4 NMR Spectroscopy

NMR experiments were run at 500.13 MHz for ¹H on a Bruker AVANCE 500 spectrometer with a Linux PC workstation, using standard 5 mm or Shigemi 3 mm tubes with susceptibility matched to solvent ²H₂O/Water sample tubes. The spectra of the polypeptide Pep8 at 0.1, 0.2, 0.4 or 1 mM sample concentrations were recorded in 50 mM sodium phosphate buffer at pH 7.4, prepared in 95% H₂O and 5% ²H₂O. Two-dimensional NMR spectra were recorded in the phase-sensitive mode using the States-TPPI method (States *et al.* 1982). All experiments were carried out using the WATERGATE pulse sequence for water suppression (Piotto *et al.* 1992) or using the excitation sculpting water suppression (Hwang et *al.* 1995) to eliminate solvent signal in H₂OPH₂O 95:5 solution. The two-dimensional COSY, TOCSY and NOESY spectra were recorded at 280 K. TOCSY spectra were recorded using a MLEV-17 spin-lock sequence (Bax *et al.* 1985) with a mixing time (τm) of 35 and 70 ms, respectively. 2D NOESY experiments were recorded at a mixing time (τm) of 100, 200 or 500 ms. The heteronuclear spectra ¹H⁻¹³C HSQC were recorded at 280 K in the same conditions. For NMR experiments with soluble RANK receptor-Fc (R&D Systems), ligand to protein ratio was ranged from 100:1 1 to 1000:1 (0.1 to 1 mM Pep8, 1 µM RANK receptor-Fc protein). Chemical shift assignments refer to internal 3-(trimethylsilyl)propionic acid-2,2,3,3-d4, sodium salt (TSP-d4). Transferred nuclear Overhauser effect (TRNOESY) spectra of Pep8 with RANK receptor-Fc protein were recorded using a mixing time (τm) of 100, 200 or 500 ms.

¹D ¹H STD NMR spectra of the polypeptide-protein mixtures were recorded at 500 MHz with 4K scans and selective saturation of protein resonances as described previously (Pons *et al.* 2011). The saturation transfer is prone to be reduced due to the low efficiency of the spin diffusion effect in low molecular weight protein (48 kDa). In order to achieve a better saturation transfer efficiency, clean STD-NMR experiments were also performed (Xia *et al.* 2010) with RF irradiation frequency values at -0.4/10.1/60 ppm (fₒₙ₁/fₒₙ₂/f_{off}). The height of Gaussian-shaped pulses was set to 200 Hz and the near ligand resonances were >500 Hz. The sensitivity enhancement in STD experiments could be achieved through optimized excitation 90° E-Burp-1 selective pulse or 90° E-Burp-1 cosine modulated selective pulse (Cutting *et al.* 2007). Interestingly, the combination of the Clean STD-NMR with 90° E-Burp-1 cosine modulated selective pulses gives better signal to noise ratio. The relative STD values observed were similar to the classical STD-NMR experiment.

Subtraction of FID values with on- and off-resonance protein saturation was achieved by phase cycling. Relative STD values were calculated by dividing STD signal intensities by the intensities of the corresponding signals in a one-dimensional ¹H NMR reference spectrum of the same sample recorded with similar parameter conditions.

### I.5 Polypeptides and reagents

All polypeptides delivered with >95% purity (HPLC) were purchased from GeneCust EUROPE (Dudelange, Luxembourg). Polypeptides were stored at -20°C until use. For *in vitro* experiments, 1 mM stock solutions were prepared in phosphate buffered saline (PBS) or cell culture medium and stored at -20°C for up to two weeks. For hydrophobic polypeptides, stock solutions containing 1-5% DMSO (v/v) were prepared. For *in vivo* experiments with polypeptide Pep8 (Pep8), a 2.5 mg/mL solution in PBS was freshly prepared and sterile filtered before use.

### I.6 Osteoclast differentiation assays

Human peripheral blood mononuclear cells (PBMCs) were isolated by centrifugation over Ficoll gradient (Sigma, Saint Quentin Fallavier, France). CD14⁺ cells were magnetically labeled with CD14 Microbeads and positively selected by MACS technology (Miltenyi Biotec, Paris, France) as described previously (Duplomb *et al.* 2008). CD11b⁺ monocytes were purified from murine bone marrow cells obtained by flushing the bone marrow from femora and tibiae of 4-week-old C57BL6 male mice, using MACS microbeads (Baud'huin *et al.* 2010). The purity of cell preparations was around 96%, as controlled by flow cytometry. Generation of osteoclasts from human CD14⁺ and murine CD11b⁺ monocytes was performed as described previously (Duplomb *et al.* 2008, Baud'huin *et al.* 2010). Briefly, purified cells were cultured in α-minimum essential medium (α-MEM, Gibco/Invitrogen, USA) with 10% fetal calf serum and 25 ng/mL human/murine macrophage colony-stimulating factor (M-CSF, R&D Systems, Abindgton, UK). After 3 days of culture, cell medium was replaced by fresh medium containing M-CSF and recombinant human RANKL (100 ng/mL, R&D system), supplemented with or without a polypeptide of interest (0.5 - 100 µM) or 50 ng/mL human OPG (R&D Systems) used as a reference inhibitor. Soluble cytokines, receptor and polypeptides were renewed every 3 days until multinucleated osteoclasts had formed. After 12 days of culture for CD14⁺ cells and 15 days for CD11b⁺ cells, osteoclasts were visualized by TRAP staining (Sigma, France) and cells formed with three or more nuclei were manually counted and analyzed statistically.

### I.7 RNA isolation and real-time PCR

Total RNA from CD14⁺ cells treated with Pep8 (25 or 50 µM) in the presence or absence of RANKL (100 ng/mL), was extracted using the Nucleospin RNA II kit (Macherey-Nagel, Düren, Germany) according to the manufacturer's instructions. Untreated cells cultured with or without RANKL served as controls. First-strand cDNA was synthesized from 5 µg total CD14⁺ RNA with ThermoScript™ RT (Invitrogen, Saint Aubin, France) and oligo(dT) primers, according to the manufacturer's recommendations. Quantitative real-time PCR (qPCR) was carried out on a Chromo4™ System (Biorad, Marnes-la-Coquette, France) with a reaction mix containing 15 - 40 ng reverse-transcribed total RNA, 300 nM primers and 2× SYBR green buffer (Biorad). Analysis was performed according to the method described by Vandesompele *et al.* (Vandesompele *et al.* 2002), using GAPDH, B2M and β-actin (ACTB) as invariant controls. The following gene-specific primers, designed with Primer 0.5 software (Whitehead Institute for Biomedical Research), were used: Cathepsin K (for) 5'-CCC AGA CTC CAT CGA CTA TCG-3', (rev) 5'-CTG TAC CCT CTG CAC TTA GCT GCC-3'; TRAP (for) 5'-AAG ACT CAC TGG GTG GCT TTG-3', (rev) 5'-GGC AGT CAT GGG AGT TCA GG-3'; NFATc1 (for) 5'-GGT CTT CGG GAG AGG AGA AA-3', (rev) 5'-TGA CGT TGG AGG ATG CAT AG-3'; GAPDH (for) 5'-TGG GTG TGA ACC ATG AGA AGT ATG-3', (rev) 5'-GGT GCA GGA GGC ATT GCT-3'; B2M (for) 5'-TTC TGG CCT GGA GGC TAT C-3', (rev) 5'-TCA GGA AAT TTG ACT TTC CAT TC-3'; ACTB (for) 5'-CCA ACC GCG AGA AGA TGA-3', (rev) 5'-CCA GAG GCG TAC AGG GAT AG-3'. PCR conditions were as follows: 30 sec at 98°C preincubation followed by 40 cycles of 15 sec at 95°C and 30 sec at 60°C (CathK, NFATc1, GAPDH, B2M, ACTB) or 30 sec at 60°C followed by 30 sec at 79°C (TRAP). Reaction products were characterized by determination of melting point (55-95°C with 0.5°C/sec).

### I.8 Western blot analysis

RAW264.7 cells were cultured in complete medium and starved for 2 hours prior to treatment with Pep8 (50 µM) in the presence or absence of RANKL (100 ng/mL). After treatment for 5, 10, 15, 30 or 60 minutes at 37°C, total cell lysates were obtained and protein concentrations were determined as described previously (Duplomb *et al.* 2008). For NF-κB pathway analysis, separate cytoplasmic and nuclear protein fractions were obtained using the NE-PER Nuclear and Cytoplasmic Extraction Kit (Thermo Scientific, UK). Proteins (40 µg) were run on 10% SDS-PAGE and transferred to Immobilon-P membranes (Millipore, Billerica, MA, USA), which were then incubated with antibodies to Akt, phospho-Akt, ERK 1/2, phospho-ERK 1/2, p38, phospho-p38, IκBα, p105, p65, p50 and RelB (Cell Signalling, Danvers, MA, USA). The labeled proteins were detected using the ECL reagent (Pierce, Rockford, IL, USA). β-Actin (total cell extract or cytoplasmic fraction), histone H3, and HDAC (nuclear fraction) were used as housekeeping proteins (Cell Signalling).

### I.9 Murine model of osteoporosis

Eight-week-old ovariectomized (OVX) female C57BL6 and non-ovariectomized control mice were purchased from Janvier (Le Genest Saint Ilsle, France). Mice were housed under pathogen-free conditions at the Experimental Therapy Unit (Faculty of Medicine, University of Nantes, France), and animal care and experimental protocols were approved by the French Ministry of Research and were done in accordance with the institutional guidelines of the French Ethical Committee and under the supervision of authorized investigators. After recovery from OVX surgery for 7 days and acclimation, the mice were randomly divided into a treatment (Pep8) and a control (OVX) group (n = 8 per group). Mice received daily subcutaneous injections of Pep8 (10 mg/kg) or the vehicle only. Non-ovariectomized (NOV) mice were included in the study as healthy controls.

During the experimental period, the body weight of the mice was monitored. No significant differences in the development of body weight were observed in ovariectomized mice during the course of the study. After treatment for five weeks, mice were anesthetized with isoflurane (2%, 1L/min) and sacrificed by exsanguination. Lumbar vertebrae and tibiae were collected for micro-CT analysis and stored at 4°C in 4% paraformaldehyde until further analysis. Additionally, internal organs (heart, liver, lungs, kidneys, spleen, intestines and thymus) of some mice (n = 3 per group) were harvested and stored for toxicity screening.

### I.10 Micro-CT analysis of bone samples

Analysis of architectural variables of tibiae and vertebrae of mice was performed using the high-resolution X-ray micro-CT system for small animal imaging SkyScan-1072 (SkyScan, Belgium). After scanning, the image data were transferred to a workstation and the proximal tibiae and the fourth lumbar vertebrae (L4) were rendered for 3-D display and calculation of the structural indices (Parfitt *et al.* 1987) using the SkyScan analysis system (CT-analyser, CT-volume, SkyScan). For trabecular bone parameters in tibiae, transverse CT slices were obtained in the region of interest in the axial direction from the trabecular bone ca. 0.1 mm below the growth plate to the mid-femur. Contours were defined and drawn close to the cortical bone. The trabecular bone was then removed and analyzed separately. Fifty slices (1 mm) at approximately 0.4 µm distal to the growth plate of the proximal ends of the tibiae were used for analysis. For the analysis of the L4 vertebrae, 120 slices (2.4 mm) were manually delineated within the vertebral body to avoid the inclusion of the superior and inferior endplates. The threshold level for the measurements was set at 55 for the analyses. The analysis of the specimens involved the following bone measurements: bone volume fraction (BV/TV, %), trabecular number (TbN), trabecular thickness (TbTh) and trabecular spacing (TbSp).

### I.11 Histological evaluation of organ toxicity

After sacrifice, organs were conserved and fixed in 4% PFA at 4°C, and embedded in paraffin. Sections (4 µm) were cut and stained with hematoxylin and eosin (Lamoureux *et al.* 2009, Baud'Huin *et al.* 2010). General morphology of organs was evaluated on each section using a DMRXA microscope (Leica, Nussloch, Germany).

### I.12 Statistical Analysis

*In vivo* data are presented as the mean ± SEM of eight animals. The significance of differences between ovariectomized mice treated with the polypeptide and vehicle-treated animals or healthy controls was determined using ANOVA and Dunnett's multiple range test. For *in vitro* data, statistical analysis was performed by use of a 2-sided Student t test; comparisons between groups were analyzed by t test (2-sided) or ANOVA for experiments with more than 2 subgroups. Probability values of *P <* 0.05 were considered statistically significant.

### II. Results

### II.1 RANKL binding to RANK allows to define a putative inhibitory region

One striking feature revealed in the structure of RANK-RANKL by the work of Liu *et al.* (2010) is the major conformational switch encompassed by RANK upon RANKL binding. Most of the bottom part of the extracellular region of RANK remains unchanged, the CRD1 and CRD2 units perform a 36° switch to come in close contact with RANKL (Liu *et al.* 2010). Although there is no strict cavity for defining a binding pocket, one can use the split resulting from the switch in the CRD2 domain to define the hinge region between the two conformations. The inventors used this cleft to define their main binding pocket for docking analysis of their polypeptide candidates (Fig 1A).

### II.2 Polypeptides of the invention targeting RANK inhibit RANKL-induced osteoclastogenesis

The inventors have designed several polypeptide inhibitors from the receptor RANK (Table 1) which were then screened for biological activity. The purified polypeptides (GeneCust) were prepared as 1 mM stock solutions in phosphate-buffered saline (PBS, pH 7.4) or cell medium. Due to the hydrophobic nature of some polypeptides, it was necessary to supplement the solvent with 1% DMSO to achieve the desired concentration. The inventors have evaluated these inhibitory polypeptides at a 50 µM concentration on osteoclastogenesis in human CD14⁺ monocytes isolated from PBMCs cultured with 25 mg/mL M-CSF and 100 ng/mL RANKL (Figure 1B). Polypeptide mimetics derived from OPG (OP3-4) and the TNF receptor (WP9QY) with a known inhibitory activity (Takasaki *et al.* 1997, Cheng *et al.* 2004), as well as the decoy receptor OPG at a concentration of 50 ng/mL were used as positive controls. Several of the designed polypeptides at a 50 µM concentration showed a moderate effect on osteoclast formation *in vitro* (Pep501 (SEQ ID NO: 26); PepA19 (SEQ ID NO: 31); PepA20 (SEQ NO: 32)), reducing the formation of TRAP-positive multinucleated cells by ca. 25-35% compared to cells treated with soluble RANKL alone.

Among the 8 new polypeptides screened in this assay, Pep8 (SEQ ID NO: 2) exhibited inhibitory activity similar to that of the RANKL-targeting mimetics OP3-4 and WP9QY, and was accordingly judged to be the most promising polypeptide inhibitor of RANK among the panel of polypeptides.

The inventors derived new polypeptides from the Pep8 sequence using molecular modeling as support for the modification. All these derived polypeptides were able to inhibit osteoclastogenesis *in vitro* and some of them had a stronger inhibition activity than Pep8 (Figure 5C). All these derived polypeptides had a sequence comprising or consisting of a sequence selected from the group consisting of SEQ ID NO: 1 and a sequence having at least 80% of identity with SEQ ID NO: 1 over the entire length of SEQ ID NO: 1.

These results demonstrate the ability of the polypeptides according to the invention to inhibit RANKL-induced osteoclastogenesis *in vitro.*

**Table 1: Polypeptides designed for binding to the RANK cleft. P8 series (Pep8 to Pep826) have a sequence comprising or consisting of a sequence selected from the group consisting of SEQ ID NO: 1 and a sequence having at least 80% of identity with SEQ ID NO: 1 over the entire length of SEQ ID NO: 1.**

| **Name of the polypeptide** | **Amino acids sequence** | **Modifications*** | **% of identity with SEQ ID NO: 1 over the entire length of SEQ ID NO: 1 of a sequence comprised in the polypeptide** | **SEQ ID NO:** |
|---|---|---|---|---|
| Pep824 | LKLCS | N-(PEG)₅ / N -(PEG)₈ | 100% | 1 |
| Pep8 | NVLKLCSGE | N-(PEG)₅ / N -(PEG)₈ | 100% | 2 |
| Pep801 | ELANVLKLCSGE | N-(PEG)₅ / N -(PEG)₈ | 100% | 3 |
| Pep802 | NVLKLCSGEAY | N-(PEG)₅ / N -(PEG)₈ | 100% | 4 |
| Pep803 | ELANVLKLCSGEAY | N-(PEG)₅ / N -(PEG)₈ | 100% | 5 |
| Pep804 | NVLKLCSGEAYR | N-(PEG)₅ / N -(PEG)₈ | 100% | 6 |
| Pep805 | NVLKLACSGE | N-(PEG)₅/N-(PEG)₈ | 83% | 7 |
| Pep806 | NVLKLCSE | N-(PEG)₅ / N -(PEG)₈ | 100% | 8 |
| Pep808 | NVLKFCSGE | N-(PEG)₅ / N -(PEG)₈ | 80% | 9 |
| Pep809 | NVIKLCSGE | N-(PEG)₅ / N -(PEG)₈ | 80% | 10 |
| Pep810 | NVLKLCHGE | N-(PEG)₅ / N -(PEG)₈ | 80% | 11 |
| Pep811 | ENVLKLCSGE | N-(PEG)₅ / N -(PEG)₈ | 100% | 12 |
| Pep812 | NALKLCSGE | N-(PEG)₅ / N -(PEG)₈ | 100% | 13 |
| Pep813 | EVLKLCSGN | N-(PEG)₅ / N -(PEG), | 100% | 14 |
| Pep814 | NALKFCSGE | N-(PEG)₅ / N -(PEG)₈ | 80% | 15 |
| Pep815 | NALKLCSGEMR | N-(PEG)₅ / N -(PEG)₈ | 100% | 16 |
| Pep816 | NALKLACSGE | N-(PEG)₅/N-(PEG)₈ | 83% | 17 |
| Pep817 | NALKLFCSGE | N-(PEG)₅/N-(PEG)₈ | 83% | 18 |
| Pep818 | NALKLACSGEMR | N-(PEG)₅/N-(PEG)₈ | 83% | 19 |
| Pep819 | NALRLCSGE | N-(PEG)₅ / N -(PEG)₈ | 80% | 20 |
| Pep820 | NALHLCSGE | N-(PEG)₅ / N -(PEG)₈ | 80% | 21 |
| Pep821 | NALFLCSGE | N-(PEG)₅ / N -(PEG)₈ | 80% | 22 |
| Pep822 | NALNLCSGE | N-(PEG)₅ / N -(PEG)₈ | 80% | 23 |
| Pep825 | YCNVLKLCSGECY | N-(PEG)₅ / N -(PEG)₈ | 100% | 24 |
| Pep826 | NALKHCSGE | N-(PEG)₅ / N -(PEG)₈ | 80% | 25 |
| Pep501 | ELASFLKISQLG | N-(PEG)₅/N-(PEG)₈ | X** | 26 |
| Pep401 | ELASFNKITQLG | N-(PEG)₅ / N -(PEG)₈ | X | 27 |
| Pep402 | ELASFNRITQLG | N-(PEG)₅ / N -(PEG)₈ | X | 28 |
| PepA17 | WLETRLTNHMELQ | N-(PEG)₅ / N -(PEG)₈ | X | 29 |
| PepA18 | AKFHGELMADQWQ | N-(PEG)₅/N-(PEG)₈ | X | 30 |
| PepA19 | NEMDLPKKSCLMN | N-(PEG)₅ / N -(PEG)₈ | X | 31 |
| PepA20 | WAARLGDPT | N-(PEG)₅ / N -(PEG)₈ | X | 32 |
| Pep1 | ELASYIIITQLG | N-(PEG)₅ / N -(PEG)₈ | X | 33 |

| | | | | |
|---|---|---|---|---|
| * Modifications evaluated to improve the peptide stability and bioavailability. ** Data not calculated. | | | | |

### II.3 Kinetic binding ability of inhibitory polypeptides

Binding of the polypeptides to RANK was studied using surface plasmon resonance (Figure 1C). Several polypeptides bound to immobilized RANK in a dose-dependent manner, presented here is Pep1 (SEQ ID NO: 33). Based on the association and dissociation kinetics obtained using a 1:1 Langmuir model for simple bimolecular interactions, the measured binding affinity (K_{d}) of Pep1 (Figure 1C) to hRANK was 20.6 µM (kₒₙ = 1,35.10¹ M⁻¹s⁻¹, k_{off} = 2,78.10⁻⁴ s⁻¹). The polypeptide of the invention Pep8, bound RANK with a K_{d} of 10.5 µM, and the apparent association constant (kₒₙ) and disassociation constant (k_{off}) rate constants were estimated to be 4.95×10²M⁻¹s⁻¹ and 5.20×10⁻³ s⁻¹ respectively.

These results demonstrate that the binding affinity (K_{d}) of the polypeptide of the invention, Pep8, to hRANK is greater than other tested polypeptides, such as Pep1.

### II.4 Pep8 inhibits RANKL-induced osteoclast formation

The inhibitory activity of the polypeptide of the invention Pep8 was then examined more in detail on RANKL-induced osteoclast formation in human CD14⁺ as well as murine CD11b⁺ cells from C57/bl6 mice. Mouse and human RANK show 77% amino acid sequence identity, therefore the inventors predicted that polypeptides generated on human RANK would also inhibit the mouse receptor homolog, and could therefore be tested in mouse models of bone loss.

Pep8 caused a dose-dependent decrease in the number of TRAP-positive multinucleated cells formed in human CD14⁺ cells (Figure 2A and B), as well as in murine cell cultures (data not shown). In the presence of 100 µM Pep8, the number of TRAP-positive multinucleated cells was 39% and 28% of the number formed in human or murine co-cultures, respectively, performed without the polypeptide. The IC₅₀ of Pep8 was 40 µM for human CD14⁺ and 30 µM for murine CD11b⁺ cells. Pep8 alone in the absence of RANKL did not modulate osteoclast development (data not shown).

Pep8 did not have intrinsic toxicity since there was no cytotoxic effect of 100 µM Pep8 on monocytes or murine macrophage-like RAW264.7 cells (data not shown), nor did the polypeptide at 100 µM exhibit any effect on mineralization of mesenchymal stem cells (MSCs), as evidenced by Alizarin red staining (data not shown).

Thus, these data suggest that Pep8 abrogates RANKL-induced osteoclastogenesis, without cytotoxicity.

To further elucidate the role of Pep8 on osteoclast differentiation, the inventors examined its effect on the gene expression of cathepsin K, TRAP, and nuclear factor of activated T cells c1 (NFATc1), all marker genes of osteoclasts. CD14⁺ monocytes were treated with M-CSF (25 ng/mL) and hRANKL (100 ng/mL) in the absence or presence of Pep8 followed by total RNA isolation.

RT- PCR showed that the mRNA expression of osteogenic markers cathepsin K, TRAP and NFATc1 was markedly reduced in the presence of Pep8 at 3 days after RANKL stimulation, and this reduction of marker expression was maintained until day 11(Figure 2C), which is consistent with the inhibitory effect of Pep8 on osteoclastogenesis.

These results demonstrate the ability of the polypeptide of the invention Pep8 to inhibit the expression of marker genes of osteoclasts.

### II.5 Pep8 inhibits RANKL-induced signaling

To further explore pathways by which Pep8 regulates osteoclast differentiation and function, the effect of the polypeptide on RANKL-induced phosphorylation of Akt, p38 and ERK was examined in RAW264.7 cells that had been exposed to 50 µM Pep8. Western blot analyses demonstrated Akt phosphorylation after 5 minutes of RANKL treatment, and Pep8 exhibited an inhibitory effect on RANKL-induced Akt phosphorylation (Figure 3A). Similarly, Pep8 inhibited phosphorylation of p38 and ERK after 15 minutes of RANKL treatment. The basal levels of pAkt and pERK were not changed in the presence of Pep8; however, Pep8 decreased phosphorylation of p38 even in the absence of RANKL stimulation (data not shown).

The inventors also examined whether Pep8 was capable of inhibiting the rapid RANKL-induced activation of NF-κB in RAW264.7 cells. The inventors found that the presence of Pep8 at 50 µM blocked the RANKL-induced degradation of IκBα and the subsequent nuclear translocation of p50 and p65 (Figure 3B). These results indicate that Pep8 inhibits the RANKL-induced activation of the classical NF-κB pathway. By contrast, treatment with 50 µM Pep8 had no effect on the expression levels of RelB in the cytoplasmic as well as in the nuclear fraction (Figure 3B).

In conclusion, the polypeptide of the invention Pep8 seems to inhibit the RANKL-induced activation only via inhibition of the classical, but not the alternative NF-κB pathway.

### II.6 Polypeptide Pep8 protects mice against ovariectomy-associated bone loss

Given Pep8's ability to inhibit RANKL-induced osteoclast differentiation and signaling *in vitro,* the inventors asked whether Pep8 might prevent bone loss *in vivo.* Estrogen deficiency in ovariectomized mice leads to accelerated bone resorption and reduced bone mineral density (Sato *et al.* 1997). The inventors therefore examined whether Pep8 *in vivo* is able to protect against OVX-induced bone loss, which requires RANKL-signaling (Cenci *et al.* 2000, Roggia *et al.* 2001).

Female C57BL/6 mice underwent ovariectomy at 8 weeks of age and were allowed to recover from surgery for one week before treatment was initiated. Mice received daily subcutaneous injections of Pep8 for 35 days at a dosage of 10 mg/kg body weight daily. *In vitro,* Pep8 actively inhibited CD11b⁺ osteoclast formation with an IC₅₀ of 30 µM (0.029 mg/mL). OVX control mice and age-matched healthy mice (NOV) were treated with the vehicle (PBS) only. Histomorphometry and µCT analysis demonstrated that treatment with Pep8 at a biologically active dose of 10 mg/kg/d induced an overall increase in trabecular bone density in ovariectomized mice compared with the vehicle-treated OVX control group (Figure 4). The effectiveness of the OVX procedure was confirmed by a reduction of bone parameters such as the bone volume fraction (BV/TV), trabecular thickness (TbTh) and trabecular number (TbN), as well as an increase in trabecular spacing (TbS) in vehicle-treated OVX mice compared to healthy NOV mice (Figure 4).

At the proximal tibia, treatment with Pep8 significantly increased trabecular BV/TV by 93% (*P* < 0.01) in ovariectomized mice. TbTh increased by 14% (*P* < 0.01) and TbN by 71% (*P* < 0.01). Pep8-treated mice also exhibited a decline in TbS (*P* < 0.01) compared with vehicle-treated mice (Figure 4A).

Similar results were obtained in lumbar vertebrae (Figure 4B). Vertebral trabecular BV/TV was lower in OVX than Pep8-treated mice (*P* < 0.05). Similarly, treatment with Pep8 increased TbN (*P* < 0.05) and reduced TbS (*P* < 0.05). TbTh was also slightly, but not statistically significantly increased in mice treated with Pep8 compared to OVX control mice.

Pep8 had no effect on the body weight in the OVX mice, indicating that a treatment at this dose had no toxic effects on animals, and this was confirmed by a histopathological evaluation of major organs (heart, lungs, liver, kidneys, spleen, skin) (Figure 6).

Taken together, these results provide evidence that the polypeptide of the invention Pep8 can, at least partially, prevent postmenopausal bone loss in an *in vivo* animal model (by targeting RANK), without toxic effects.

### II.7 STD NMR study of the interaction between Pep8 RANK

A Saturation Transfer Difference (STD-NMR) experiment was performed with hRANK and Pep8 in a phosphate buffer (Figure 5A). Pep8 spectra were measured first without the protein, and the polypeptide remained stable under the experimental NMR conditions throughout the length of the experiment (2 weeks). hRANK was also studied in the same phosphate buffer, and increasing concentrations of Pep8 (with a concentration ratio from 100:1 to 1000:1) were added to the NMR tube to analyze the Pep8-hRANK interaction. The spectra of Pep8 bound to RANK differed significantly from that of the soluble unbound Pep8, in a dose-dependent manner, proportional to the addition of Pep8 to the NMR tube. The Pep8/hRANK interaction was analyzed more than two weeks after adding the polypeptide to the mix, and no additional visible shifts in the bound spectra were observed, apart from those induced by the binding of Pep8 to its target RANK. The STD experiment revealed that the 5 central amino acids of Pep8 were in direct interaction with hRANK (L₃K₄L₅C₆S₇) with three receiving 100 % of the saturation (L₃K₄L₅). C₆S₇ had a relative transfer ratio of 60 % and G₈E₉ received lower transfers. N₁V₂ were not visible in the spectra, which is typical in this type of experiment, but both terminal NH of N₁ were detectable with a signal close to the signal of G₈E₉. These results indicate that Pep8 forms a tight interaction with hRANK and that most of these interactions are concentrated in the core 5 amino acids (L₃K₄L₅C₆S₇, at least for the backbone NH). These interactions define a continuous patch of interactions throughout hRANK, which is in perfect agreement with the positions inferred from the molecular modeling analysis (Figure 1A and 5B).

These results provide clear evidences of the direct interaction of the polypeptide of the invention Pep8 with hRANK, in a conformation which is in agreement with the modeled interaction.

### BIBLIOGRAPHIC REFERENCES

Aoki K, Saito H, Itzstein C, et al. (2006). A TNF receptor loop peptide mimic blocks RANK ligand-induced signaling, bone resorption, and bone loss. J Clin Invest 116: 1525-1534.
Baron R, Ferrari S, and Russell R. (2011). Denosumab and bisphosphonates: Different mechanisms of action and effects. Bone 48: 677-692.
Baud'Huin M, Duplomb L, Téletchéa S, et al (2009). Factor VIII-von Willebrand factor complex inhibits osteoclastogenesis and controls cell survival J Biol Chem 284: 31704-13.
Baud'Huin M, Renault R, Charrier C, et al (2010). Interleukin-34 is expressed by giant cell tumours of bone and plays a key role in RANKL-induced osteoclastogenesis. J Pathol 221 (1): 77-86.
Bax A and Davis DG (1985). MLEV-17-based two-dimensional homonuclear magnetization transfer spectroscopy, J Magn Reson 65: 355-60.
Boyce BF, Xing L, Yao Z, et al. (2006). Future Anti-Catabolic Therapeutic Targets in Bone Disease. Annals of the New York Academy of Sciences 1068: 447-457.
Brooks BR, Bruccoleri RE, Olafson BD, et al (1983). CHARMM: A Program for Macromolecular Energy, Minimization, and Dynamics Calculations, J Comp Chem 4: 187-217,
Cenci S, Weitzmann MN, Roggia C, et al (2000). Estrogen deficiency induces bone loss by enhancing T-cell production of TNF-a. J Clin Invest 106: 1229-37.
Cheng X, Kinosaki M, Takami M, et al (2004). Disabling of Receptor Activator of Nuclear Factor-kB (RANK) Receptor Complex by Novel Osteoprotegerin-like Peptidomimetics Restores Bone Loss in Vivo. J Biol Chem 279 (9): 8269-77.
Cutting B, Shelke SV, Dragic Z, et al (2007). Sensitivity enhancement in saturation transfer difference (STD) experiments through optimized excitation schemes. Magn Reson Chem 45 (9): 720-24.
Dai X, Ma W, He X, et al. (2011). Review of therapeutic strategies for osteosarcoma, chondrosarcoma, and Ewing's sarcoma. Med. Sci. Monit. 17: RA177-RA190.
Demchenko YN, and Kuehl WM. (2010). A critical role for the NFkB pathway in multiple myeloma. Oncotarget 1: 59-68.
Derossi D, Joliot AH, Chassaing G, and Prochiantz A. (1994). The third helix of the Antennapedia homeodomain translocates through biological membranes. Journal of Biological Chemistry 269: 10444-10450.
Duplomb L, Baud'huin M, Charrier C, et al. (2008). Interleukin-6 inhibits receptor activator of nuclear factor-κB ligand-induced osteoclastogenesis by diverting cells into the macrophage lineage: key role of Serine727 phosphorylation of signal transducer and activator of transcription 3. Endocrinology 149: 3688-97.
Duheron V, Hess E, Duval M, et al. (2011). Receptor activator of NF-κB (RANK) stimulates the proliferation of epithelial cells of the epidermo-pilosebaceous unit. PNAS 108: 5342-5347.
Gonzalez-Suarez E, Jacob AP, Jones J, et al. (2010). RANK ligand mediates progestin-induced mammary epithelial proliferation and carcinogenesis. Nature 468: 103-107.
Hansen KE, Wilson HA, Zapalowski C, et al. (2011). Uncertainties in the prevention and treatment of glucocorticoid-induced osteoporosis. Journal of Bone and Mineral Research 26: 1989-1996**.**
Hwang TL and Shaka AJ (1995). Water Suppression That Works - Excitation Sculpting Using Arbitrary Wave-Forms and Pulsed-Field Gradients, J Magn Reson Ser A 112: 275-79.
Lamoureux F, Picarda G, Garrigue L, et al (2009). Glycosaminoglycans as potential regulators of osteoprotegerin therapeutic activity in osteosarcoma. Cancer Res 69: 526-36.
Jones DH, Nakashima T, Sanchez OH, et al. (2006). Regulation of cancer cell migration and bone metastasis by RANKL. Nature 440: 692-696.
Lee BL, Higgins MJ, and Goss PE. (2011). Denosumab and the current status of bone-modifying drugs in breast cancer. Acta Oncologica, 1-11.
Lien S, and Lowman HB. (2003). Therapeutic peptides. Trends in Biotechnology 21: 556-562.
Liu C, Walter TS, Huang P, et al (2010). Structural and Functional Insights of RANKL-RANK Interaction and Signaling. J Immunol 184: 6910-19.
May MJ, D'Acquisto F, Madge LA, et al. (2000). Selective Inhibition of NF-κB Activation by a Peptide That Blocks the Interaction of NEMO with the IκB Kinase Complex. Science 289: 1550-1554.
Mori K, Berreur M, Blanchard F, et al. (2007). Receptor activator of nuclear factor-kappaB ligand (RANKL) directly modulates the gene expression profile of RANK-positive Saos-2 human osteosarcoma cells. Oncol Rep 18: 1365-1371.
Needleman SB, and Wunsch CD. (1970). A general method applicable to the search for similarities in the amino acid sequence of two proteins. Journal of Molecular Biology 48: 443-453.
Parfitt AM, Drezner MK, Glorieux FH, et al (1997). Bone histomorphometry: standardization of nomenclature, symbols, and units. Report of the ASBMR Histomorphometry Nomenclature Committee. J Bone Miner Res 2 (6): 595-610.
Petersen TN, Brunak S, von Heijne G, et al. (2011). SignalP 4.0: discriminating signal peptides from transmembrane regions. Nat Meth 8: 785-786.
Piotto M, Saudek V and Sklenar V (1992). Gradient-tailored excitation for single-quantum NMR spectroscopy of aqueous solutions. J Biomol NMR 2 (6): 661-65.
Pons J, Tanchou V, Girault JP, Bertho G, Evrard-Todeschi N (2011). NMR applications for identifying β-TrCP protein-ligand interactions. Med Chem 11 (4): 283-97. Review.
Roggia C, et al. (2001). Up-regulation of TNF-producing T cells in the bone marrow: a key mechanism by which estrogen deficiency induces bone loss in vivo. Proc Natl Acad Sci USA 98: 13960-65.
Sato M, Zeng GQ and Turner CH (1997). Biosynthetic human parathyroid hormone (1-34) effects on bone quality in aged ovariectomized rats. Endocrinology 138: 4330-4337.
Santini D, Perrone G, Roato I, et al. (2011). Expression pattern of receptor activator of NκKB (RANK) in a series of primary solid tumors and related bone metastases. Journal of Cellular Physiology 226: 780-784.
Schramek D, Leibbrandt A, Sigl V, et al. (2010). Osteoclast differentiation factor RANKL controls development of progestin-driven mammary cancer. Nature 468: 98-102.
Smith HS. (2011). Painful osseous metastases. Pain Physician 14: E373-E403.
States DJ, Haberkorn RA, Ruben DJ (1982). A two-dimensional nuclear Overhauser experiment with pure absorption phase in four quadrants. J Magn Res 48: 286-92.
Ta HM, Nguyena GTT, Jinb HM, et al (2010). Structure-based development of a receptor activator of nuclear factor-κB ligand (RANKL) inhibitor peptide and molecular basis for osteopetrosis. PNAS 107 (47): 20281-86.
Takasaki W, Kajino Y, Kajino K, et al (1997). Structure-based design and characterization of exocyclic peptidomimetics that inhibit TNFα binding to its receptor. Nat Biotech 15: 1266-70.
Tanaka S, Nakamura K, Takahasi N, et al., (2005). Role of RANKL in physiological and pathological bone resorption and therapeutics targeting the RANKL-RANK signaling system. Immunological Reviews 208: 30-49.
Theoleyre S, Wittrant Y, Tat SK, et al. (2004). The molecular triad OPG/RANK/RANKL: involvement in the orchestration of pathophysiological bone remodeling. Cytokine Growth Factor Rev 15: 457-475.
Toes REM, van der Voort EIH, Schoenberger SP, et al. (1998). Enhancement of Tumor Outgrowth Through CTL Tolerization After Peptide Vaccination Is Avoided by Peptide Presentation on Dendritic Cells. The Journal of Immunology 160: 4449-4456.
Vandesompele J, De Preter K, Pattyn F, et al. (2002). Accurate normalization of real-time quantitative RT-PCR data by geometric averaging of multiple internal control genes. Genome Biol 3: RESEARCH0034
Whyte LS, Ryberg E, Sims NA, et al. (2009). The putative cannabinoid receptor GPR55 affects osteoclast function in vitro and bone mass in vivo. PNAS 106: 16511-16516.
Wittig JC, Bickels J, Priebat D, et al. (2002). Osteosarcoma: a multidisciplinary approach to diagnosis and treatment. Am Fam Physician 65: 1123-1132.
Wu G, Robertson DH, Brooks CL, et al (2003). Detailed analysis of grid-based molecular docking: A case study of CDOCKER-A CHARMm-based MD docking algorithm. J Comp Chem 24 (13): 1549-62.
Xia Y, Zhu Q, Jun KY, Wang J, Gao X (2010). Clean STD-NMR spectrum for improved detection of ligand-protein interactions at low concentration of protein. Magn Reson Chem 48 (12): 918-24.
Xu M, Choudhary S, Voznesensky O, et al (2010). Basal bone phenotype and increased anabolic responses to intermittent parathyroid hormone in healthy male COX-2 knockout mice. Bone 47 : 341-352

## Claims

1. An isolated polypeptide having a sequence of up to 20 amino acids, wherein said sequence comprising or consisting of a sequence selected from the group consisting of:
- SEQ ID NO: 1; and
- a sequence having at least 80% of identity with SEQ ID NO: 1 over the entire length of SEQ ID NO: 1;
for use as a medicament in the treatment and/or prevention of a bone resorptive disease.

2. The isolated polypeptide for use according to claim 1, wherein said bone resorptive disease is selected from the group consisting of:
- osteoporosis, osteolytic bone disease, primary bone cancers, secondary bone cancers, periodontal disease and rheumatoid arthritis.

3. The isolated polypeptide for use according to claim 1 or 2, wherein said polypeptide has a sequence comprising or consisting of a sequence selected from the group consisting of:
- SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25.

4. The isolated polypeptide for use according to claim 3, wherein said polypeptide has a sequence consisting of a sequence selected from the group consisting of:
- SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 and SEQ ID NO: 25.

5. The isolated polypeptide for use according to any one of claims 1 to 4, wherein said polypeptide contains at least one biochemical modification selected from the group consisting of pegylation, acetylation, formylation, myristic acid addition, palmytoylation, benzyloxycarbonylation, amidation, succinylation, glycosylation, in particular pegylation.

6. An isolated polypeptide as defined in claim 3 or 4.

7. The isolated polypeptide according to claim 6, wherein said polypeptide has the amino acid sequence set forth in SEQ ID NO: 2.

8. An isolated nucleic acid molecule encoding an isolated polypeptide as defined in claim 6 or 7.

9. A vector comprising a nucleic acid molecule as defined in claim 8.

10. A host cell comprising a nucleic acid molecule as defined in claim 8 or a vector as defined in claim 9.

11. A pharmaceutical composition comprising at least one compound selected from the group consisting of an isolated polypeptide as defined in claim 6 or 7, an isolated nucleic acid molecule as defined in claim 8 and a vector as defined in claim 9.

12. An isolated polypeptide as defined in claim 6 or 7 or an isolated nucleic acid molecule as defined in claim 8 or a vector as defined in claim 9 for use as a medicament.

13. A combination product comprising:
- at least one compound selected from the group consisting of an isolated polypeptide as defined in any one of claims 1 to 5, an isolated nucleic acid molecule encoding said polypeptide and a vector comprising said nucleic acid molecule; and
- another bone anti-resorptive agent;
for simultaneous, separate or sequential use as a medicament.

14. The combination product for use according to claim 13, wherein said another bone anti-resorptive agent is selected from the group consisting of:
- anabolism enhancers, in particular selected from the group consisting of parathyroid hormone, BMP2, vitamin D, anti-inflammatory agents; and
- catabolism inhibitors, in particular selected from the group consisting of bisphosphonates, cathepsin K inhibitors, p38 inhibitors, JNK inhibitors, IKK inhibitors, NF-κB inhibitors, calcineurin inhibitors, NFAT inhibitor, PI3K inhibitor.

15. The combination product according to claim 13 or 14, for use in the treatment and/or prevention of a bone resorptive disease.
